# EUROPEAN PATENT APPLICATION

(11) **EP 2 292 895 A2**
(43) Date of publication of application: **09.03.2011**
(21) Application number: 10075746.7
(22) Date of filing: 23.08.2007
(51) Int. Cl.: E21B 49/08

(54) **A device and method for analyzing light chemical compounds**

(62) Divisional of application: 07291037.5
(71) Applicant: Services Pétroliers Schlumberger, 75007 Paris (FR); Schlumberger Technology B.V., 2514 JG The Hague (NL); Schlumberger Holdings Limited, Tortola (VG); PRAD Research and Development Limited, Road Town, Tortola (VG)
(72) Inventor: The designation of the inventor has not yet been filed
(74) Representative: Vandermolen, Mathieu

(57) **Abstract**

An analyzing device to analyze light chemical compounds comprises:
- an injector (3) to inject a fluid sample from a flow-line (1) in the analyzing device,
- a low pressure chamber (4) comprising a low pressure piston (5), the low pressure chamber (4) receiving the fluid sample from the injector (3),
- a high pressure chamber (11) comprising a high pressure piston (12), the high pressure chamber (11) receiving the fluid sample from the low pressure chamber (4),
- an analyzing sensor (7) coupled to one or more control sensors (8, 9), and receiving the fluid sample from the high pressure chamber (11), and
- a controller (14) receiving measurements from the analyzing sensor (7) and the control sensors (8, 9) and commanding operations of the injector (3), the low pressure piston (5), and the high pressure piston (12) such that the low pressure piston (5) pushes the fluid sample toward the analyzing sensor (7) and the high pressure chamber (11) in sweeping a determined pressure range encompassing an optimal pressure, the high pressure piston (12) pushes the fluid sample back to the flow line (1) after the fluid sample has been transferred to the high pressure chamber (11).

## Description

### FIELD OF THE INVENTION

The invention relates to a device and a method used for analyzing light chemical compounds of fluids flowing in a borehole penetrating geological formations, or fluids present in the geological formations. The invention finds a particular application in the oilfield industry.

### BACKGROUND OF THE INVENTION

The document GB 2 344 365 describes a sampling tool for trapping and concentrating volatile component of a well effluent on a solid material for further analysis. Such a technique and sampling tool has the drawbacks that the collected samples must be sent at the surface for analysis in specialized laboratories. Thus, the measurements are made off-line. This is most of the time incompatible with the current trend of construction of hydrocarbon production; processing and transportation facilities where it is desirable to quickly know whether corrosive compounds (e.g. H₂S) are contained within the formation fluids in order to select the appropriate metal used for the various pipes.

The documents US 7,025,138 and US 2006/0243603 describe apparatuses for in-situ monitoring of the corrosive compounds (e.g. H₂S) contained within the downhole formation fluids. Such techniques and apparatuses have the drawbacks that the sample pressure is imposed by the well pressure. Thus, the sample phase is not controlled and may be multiphase, e.g. a mixture of liquid water, oil, and gas. Generally, the presence of liquid phase in the sample limits the accuracy of measurements on light compounds. Even for pure gas effluents, the measurement dynamic range (ratio between the highest and lowest concentrations that can be measured) is limited.

WO 02/084334 describes a logging while drilling and wire line system for analyzing the concentration of carbon dioxide or another substance, in a sample down hole in a bore hole. A chamber is filled with a sample that may be fluid, or condensate and gas. The interior chamber volume is selectably expandable for decompression of the sample. The sample may alternately be decompressed by allowing a sample at formation pressure to enter the chamber at a lower pressure, thereby decompressing the sample. A sensor measures the absorbance, transmittance or attenuated total reflectance of the infrared light. Mid and near infrared light is utilized to identify carbon dioxide, water and a plurality of hydrocarbons. A wiper cleans the transmitter and sensor between readings to reduce measurement error caused by fluid sample contamination.

US 2006/0144126 describes methods and systems for analyzing samples, such as gas samples. One method comprises providing a gas sample, increasing pressure applied to the gas sample to compress the sample to a smaller volume and provide a pneumatically focused gas sample, and analyzing the pneumatically focused gas sample using any of a variety of analytical techniques. Also disclosed are systems for gas analysis, including systems for analysis of pneumatically focused, and thereby concentrated, gas samples and for analysis of particulate matter in gas samples.

US 5,147,561 describes a sampling device for sealed positioning within the casing of a well containing a stripping chamber for stripping a ground water sample of its volatile compounds at or near the point of collection. In one embodiment, the stripping chamber includes a piston which is raised above the top surface of the sample to create a head space, and an injector for injecting inert gas into the sample, causing the volatile compounds to be released into the head space, whereupon they exit the chamber via a vapor outlet duct leading out of the well. In a second embodiment, the stripping chamber includes a tube having a sidewall formed of a semi-permeable membrane, and an injector which injects an inert gas into the bore of the tube. The device may also include a sensor mounted within the stripping chamber, and a valve for directing a calibration gas into the chamber.

### SUMMARY OF THE INVENTION

It is an object of the invention to propose a device and a method used for analyzing light chemical compounds of fluids flowing in a borehole penetrating geological formations, or fluids present in the geological formations that overcome at least one of the drawbacks of the prior art measuring apparatuses and methods.

According to an aspect, the invention relates to an analyzing device to analyze light chemical compounds comprising:
- an injector to inject a fluid sample from a flow-line in the analyzing device,
- a low pressure chamber comprising a low pressure piston, the low pressure chamber receiving the fluid sample from the injector,
- an analyzing sensor coupled to a plurality of control sensors, and receiving the fluid sample from the low pressure chamber,
- a high pressure chamber comprising a high pressure piston, the high pressure chamber receiving the fluid sample from the analyzing sensor, and
- a controller receiving measurements from the analyzing sensor and the control sensors and commanding operations of the injector, the low pressure piston, and the high pressure piston such that the low pressure piston pushes the fluid sample toward the analyzing sensor in sweeping a determined pressure range encompassing an optimal pressure, the high pressure piston pushes the fluid sample back to the flow line after the fluid sample has been transferred to the high pressure chamber.

The analyzing sensor may further comprises a temperature regulator, operations of which being commanded by the controller.

The injector may comprise a sliding valve fitted in a body, the sliding valve comprising a sampling chamber, at least one seal and an output port, the body being coupled to the flow-line and comprising a bypass groove.

Optionally, the sampling chamber may comprise walls that are textured such as to enhance the trapping of liquid in the sampling chamber.

The device may further comprise a first check valve between the high pressure chamber and the sensor to prevent the fluid sample from uncontrolled exit of the high pressure chamber.

The device may further comprise a second check valve between the high pressure chamber and the flow-line to prevent the fluid of the flow-line to enter the high pressure chamber through an output port.

The device may further comprise a filter between the low pressure chamber and the analyzing sensor to trap at least a portion of a liquid phase contained in the fluid sample.

The sensor may be an optical absorption/transmission spectroscopy sensor comprising a housing defining an optical cell, an inlet port, an output port, and an optical path between a light source and a detector coupled to the optical cell by respective wave guides. Alternatively, the sensor may be a gas chromatography sensor.

The control sensors may comprise a pressure sensor and a temperature sensor.

The temperature regulator may be a heating coil to adjust the temperature within the analyzing sensor at an optimal temperature value.

Advantageously, the high pressure piston has a smaller cross section than the low pressure piston.

According to a further aspect, the invention relates to a method to perform downhole analysis of light chemical compounds comprising the steps of:
- flashing a fluid sample from a flow-line in order to depressurize the fluid sample so that light compound of the fluid sample become gaseous, and transferring the fluid sample in a chamber of an analyzing sensor,
- sweeping a range of pressure until the pressure measured in the chamber of the sensor is optimal,
- measuring at least one characteristic related to at least one compound of the fluid sample while continuously controlling the fluid sample pressure and temperature,
- draining the fluid sample out of the chamber of the sensor, and
- discarding the fluid sample in the flow-line.

Optionally, the method further comprises the step of heating the fluid sample in the sensor chamber until the temperature measured in the chamber of the sensor is optimal.

The pressure may be adjusted from a low value to a high value while measuring at least one characteristic related to at least one compounds of the fluid sample such as a range of concentration of the compound is swept, the optimal pressure corresponding to a light compound concentration associated with the lowest measurement error.

The optimal temperature may correspond to substantially the flow-line temperature.

Thus, the invention enables depressurizing the sample so that light compounds of the fluid sample become gaseous and preventing the liquid phase from reaching the sensor. Further, the invention enables controlling the sample pressure and temperature when the measurements are taken. As a consequence, with the invention, the capacity of the analyzing device to provide accurate measurements related to light chemical compounds is not limited, and independent on the well pressure. In particular, the invention is adapted to perform downhole analysis of light chemical compounds contained in pressurized effluents of hydrocarbon well. Furthermore, once analyzed, the sample is discarded downhole and the sensor chamber is automatically "regenerated" for further measurements.

These and other aspects of the invention will be apparent from and elucidated with reference to the embodiments described hereinafter.

### BRIEF DESCRIPTION OF THE DRAWINGS

The present invention is illustrated by way of example and not limited to the accompanying figures, in which like references indicate similar elements:
FIG. 1 schematically shows a typical onshore hydrocarbon well location illustrating a particular application of the device for analyzing light chemical compounds of the invention;
FIG. 2 is a functional diagram illustrating the device for analyzing light chemical compounds of the invention;
FIGS. 3 and 4 are cross-section views showing in details the injector of FIGS. 2 and illustrating the injector operation;
FIGS. 5, 6 and 7 are cross-section views showing in details the first check valve of FIG. 2 and illustrating the check valve operation;
FIG. 8 is a functional diagram illustrating an embodiment of a sensor of FIG. 2 in the form of an absorption spectroscopy sensor; and
FIG. 9 is a diagram illustrating the sequence of steps of the method for analyzing light chemical compounds of the invention.

### DETAILED DESCRIPTION OF THE INVENTION

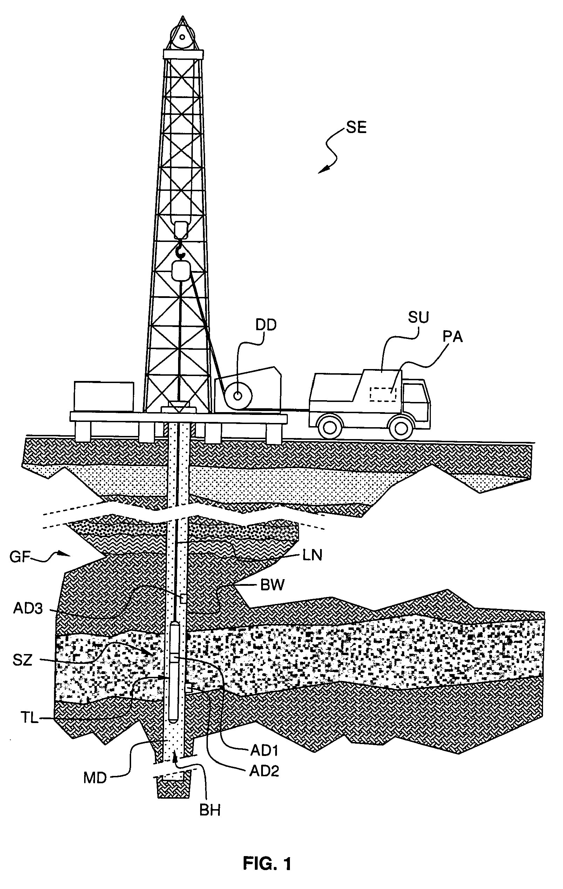

FIG. 1 schematically shows a typical onshore hydrocarbon well location and surface equipments SE above hydrocarbon geological formations GF after drilling operations have been carried out. At this stage, i.e. before a casing string is run and before cementing operations are carried out, the wellbore is a borehole BH filled with a fluid mixture MD. The fluid mixture MD is typically a mixture of drilling fluid and drilling mud. In this example, the surface equipments SE comprise an oil rig and a surface unit SU for deploying a logging tool TL in the well-bore. The surface unit may be a vehicle coupled to the logging tool by a line LN. Further, the surface unit comprises an appropriate device DD for determining the depth position of the logging tool relatively to the surface level. The logging tool TL comprises an analyzing device AD1 that performs chemical analysis of the fluid contained within the geological formation GF or flowing into the borehole BH. The logging tool may comprise various other sensors and may provide various measurement data related to the hydrocarbon geological formation GF and/or the fluid mixture DM (e.g. conductivity, resistivity, etc...). These measurement data are collected by the logging tool TL and may be transmitted to the surface unit SU by any known technique, or otherwise stored in the logging tool memory for subsequent processing when the memory is returned to the surface. The surface unit SU comprises appropriate electronic and software arrangements PA for processing, analyzing and storing the measurement data provided by the logging tool TL. Once the logging tool TL is positioned at a desired depth, characteristic parameter of a selected zone SZ of the formation, or of the fluid in the vicinity of the logging tool can be measured. Such a measurement can be repeated for other azimuth and other depth. Alternatively, in order to permanently monitor the chemical compounds present downhole, the analyzing device AD2 may be positioned within the geological formation GF, for example in the selected zone SZ of the formation. As another alternative, the analyzing device AD3 may be positioned on the borehole wall.

Though not shown, those versed in the art know that the logging apparatus of FIG. 1 can also be adapted into a logging-while-drilling tool by mounting the logging tool TL on a drill collar. More precisely, a typical logging-while-drilling tool is incorporated into a bottom-hole assembly attached to the end of a drill string with a drill bit attached at the extreme end thereof. Measurements can be made either when the drill string is stationary or rotating. The measurement data that are collected by the logging tool may be transmitted by means of the known mud pulse technique to the surface unit coupled to a mud pulse receiver.

Further, the analyzing device may also be positioned in a cased well (not shown).

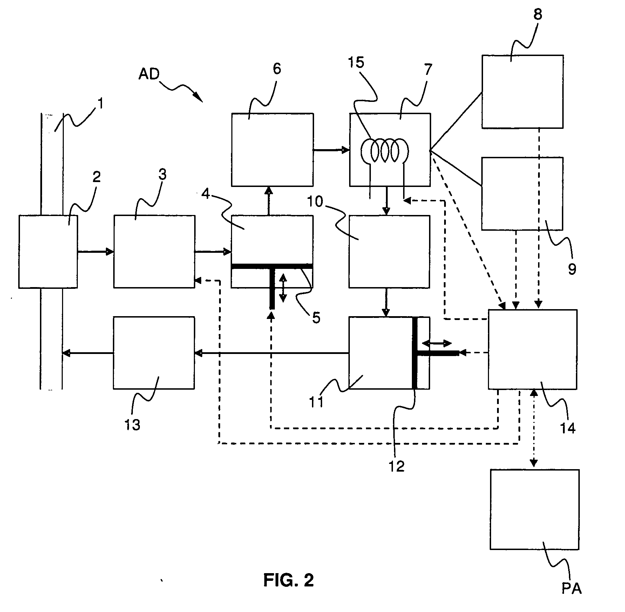

FIG. 2 is a functional diagram illustrating the device for analyzing light chemical compounds of the invention. The device for analyzing light chemical compounds AD comprises an injector 3, a low pressure chamber 4 and piston 5, an analyzing sensor 7, a plurality of control sensors 8, 9, a temperature regulator 15, a first check valve 10, a high pressure chamber 11 and piston 12, a second check valve 13, and a controller 14.

A fluid to be analyzed flows through a flow line 1. The flow line 1 schematically represents either the borehole BH or the vicinity of the analyzing device AD where a fluid is present. The fluid is sampled at an intake 2 of the injector 3. The injector 3 flashes the sample of fluid to pressure lower than the pressure of the fluid in the flow line 1. For application in the oilfield domain, the intake pressure is the well pressure, e.g. typically from around 70 bars to around 700 bars. Then, the sample is injected in the low pressure chamber 4. As an example, the sample injected in the low pressure chamber is around 0.1 ml under pressure. After injection, the pressure in the low pressure chamber is from around 0.2 bar to around 7 bar. The low pressure chamber 4 comprises the low pressure piston 5. As an example, the low pressure chamber volume is about 10 ml. When operated, the low pressure piston 5 pushes the sample toward the analyzing sensor 7.

Advantageously, the volume of the connecting tubes (not shown) between the different elements and the sensor, that are upstream the check valve 10, are smaller than the volume of the low pressure chamber 4. This enables a good efficiency of the fluid transfer from the low pressure chamber 4 to the high pressure chamber 11.

The analyzing device AD may further comprise a filter 6 between the low pressure chamber 4 and the analyzing sensor 7. Advantageously, the filter 6 is a liquid filter that traps the remaining liquid phase from the sample. For example, the liquid filter may be designed to maintain the liquid phase away from the sensor by trapping it. It may be made of a fritted inert material such as porous ceramic having a pore size of the order of 10 pm. Through viscosity effect, the liquid in the sample moves slowly in the filter while being bypassed by the gaseous phase of lower viscosity than the liquid phase.

The analyzing sensor 7 is coupled to the plurality of control sensors 8, 9. Advantageously, the pressure and the temperature of the sample in the sensor 7 are monitored via a pressure sensor 8 and a temperature sensor 9, respectively. The low pressure piston 5 moves in order to adjust the pressure within the analyzing sensor 7 so that an optimal pressure is reached. As an example, the pressure during measurement is around 10 bar and is increased in order to reach 100 bar. Advantageously, the analyzing sensor 7 may also comprise a temperature regulator, for example a heating coil 15. The heating coil 15 heats the sensor in order to adjust the temperature within the analyzing sensor 7 so that an optimal temperature is reached.

Once the measurement process within the sensor is finished, the low pressure piston 5 continues to move so as to reach a full extension course. As a consequence, the sample is pushed toward the high pressure chamber 11, through the first check valve 10. The high pressure chamber 11 comprises the high pressure piston 12. As an example, the high pressure chamber volume is about 1 ml. When the low pressure piston 5 reaches the full extension, substantially all the sample has been transferred to the high pressure chamber. When operated, the high pressure piston 12 pushes the sample back to the flow line 1, through the second check valve 13.

Advantageously, the volume of the connecting tubes between the different elements, that are upstream the high pressure chamber 11, are smaller than the volume of the high pressure chamber 11. This enables a good efficiency of the fluid transfer from the high pressure chamber 11 back to the flow line 1.

The actuation of the low pressure 5 and high pressure 12 pistons may be hydraulic or electrical. Advantageously, the two pistons have different cross section to minimize the range of force which is required to actuate them. For example, the high pressure piston 12 may have a smaller cross section than the low pressure piston 5.

The controller 14 is coupled to and receives measurements from the sensor 7, the pressure sensor 8 and the temperature sensor 9. The controller 14 is coupled to and commands the operations of the injector 3, the low pressure piston 5, the heating coil 15 and the high pressure piston 12. The controller 14 may be further coupled to the processing arrangement PA of the surface equipment or to a memory (not shown) for real-time or subsequent interpretation of the measurements.

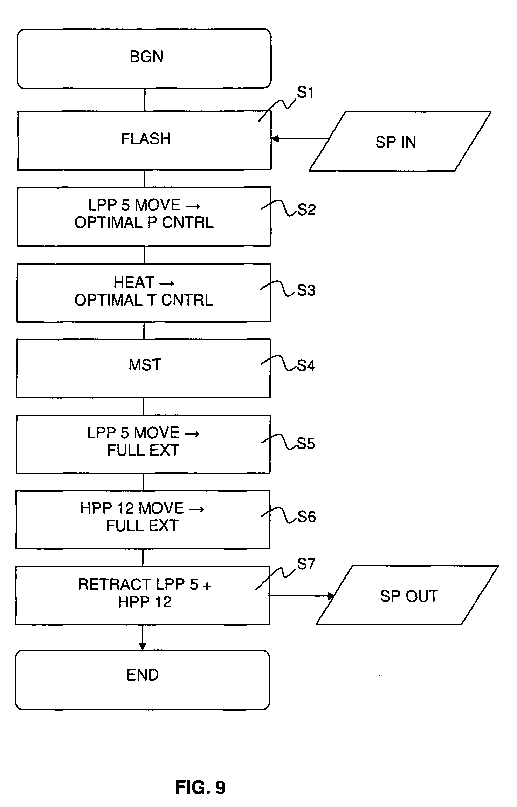

The sequence of operations that have been described hereinbefore, and that will be further described in relation with FIG. 9 is controlled by the controller 14.

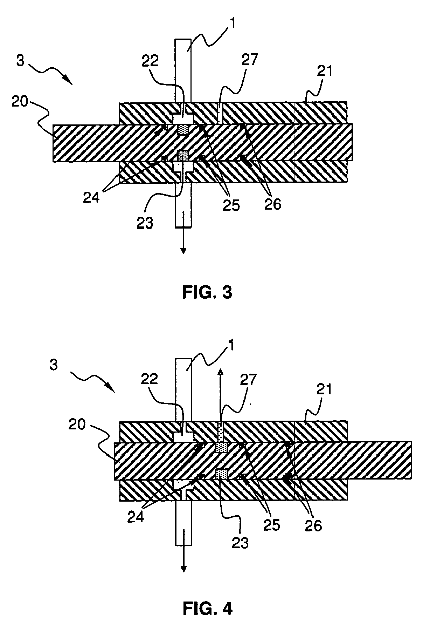

FIGS. 3 and 4 are cross-section views showing in details the injector 3 of FIGS. 2 and illustrating the injector 3 operation.

The injector comprises a sliding valve 20, a body 21, a bypass groove 22, a sampling chamber 23, various seals 24, 25, and 26, and an output port 27. The sliding valve 20 is fitted into the body 21. The intake and injection are performed by the movement of the sliding valve 20 relatively to the body 21. The sliding valve 20 moves between two positions.

In a first position of the sliding valve depicted in FIG. 3, a volume of fluid forming a sample volume is defined by the sampling chamber 23. Advantageously, the sampling chamber is defined by an annular groove in the sliding valve 20. In the first position, the sampling chamber 23 is always in contact with the effluents flowing in the flow line 1. Thus, the volume of fluid present in the sampling chamber 23 is continuously renewed by the flow. As a consequence, a good representativeness of the sample with respect to the effluent composition is substantially always guaranteed.

In a second position of the sliding valve depicted in FIG. 4, the sample volume is isolated from the flow line 1 and put in communication with the low pressure chamber 4 via the output port 27. In this position, the bypass groove 22 bypasses the sliding valve 20 and enables the flow line continuing flowing through the injector 3. When the sliding valve 20 reaches the second position, the sample volume is flashed towards the low pressure chamber 4.

Advantageously, the three seals 24, 25 and 26 are O-ring seals. Two O-ring seals are positioned on each side of the annular sampling chamber 23. The third O-ring seal is positioned such that, in the second position, the output port 27 is encompassed on each side by such an O-ring seal. They insure a good isolation between, on the one side, the flow line 1 and the bypass groove 22, and, on the other side, the output port 27. With this configuration, a good isolation is maintained even when a high pressure differential exists between the flow line 1 and the low pressure chamber 4.

As an alternative, the wall of the annular sampling chamber 23 of the sliding valve 20 may be textured such as to enhance the trapping of liquid in the sampling chamber 23. The textured wall maximizes the surface to which the flowing fluid is exposed. The capillary forces retain the liquid on the wall surface of the sampling chamber 23 while the gas is expelled (due to the flashing process) toward the low pressure chamber 4.

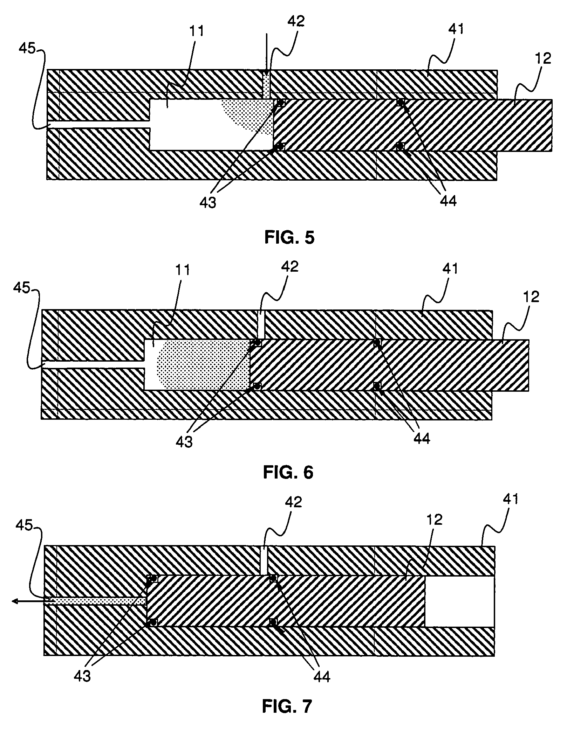

FIGS. 5, 6 and 7 are cross-section views showing in details the check valve 10 of FIG. 2 and illustrating the check valve operation.

The first check valve 10 has the function of preventing the liquid which has entered the high pressure chamber 11 from exiting it, when the high pressure piston 12 is actuated.

Advantageously, the check valve may be implemented on the high pressure piston which acts as a sliding valve. The check valve comprises a body 41, the high pressure piston 12, an inlet port 42, an outlet port 45 and two seals 43 and 44.

The high pressure piston 12 may move continuously in the high pressure chamber 11 from a fully retracted position depicted in FIG. 5, followed by a middle position depicted in FIG. 6, to a fully extended position depicted in FIG. 7.

During the high pressure piston operation, the sample is compressed and pushed from the high pressure chamber 11 toward the flow line 1 via the outlet port 45.

When the high pressure piston 12 is in the fully retracted position (FIG. 5), the inlet port 42 is in communication with the high pressure chamber 11. The high pressure piston 12 starts moving as soon as the sample has been transferred from the chamber of the sensor 7 into the high pressure chamber 11. Then, the chamber inlet port 42 is getting closed (FIG. 6), thus, preventing the sample fluid from returning back in the sensor 7 through the inlet port 42. When the high pressure piston 12 is in the fully extended position (FIG. 7), the sample is flushed out of the analyzing device AD into the flow line 1.

Advantageously, the two seals 43 and 44 are O-ring seals. The two O-ring seals are positioned such that, in the fully extended position (FIG. 7), the inlet port 42 is encompassed on each side by such an O-ring seal. With this configuration, a good isolation is maintained even when a high pressure differential exists between the flow line 1 and the chamber of the sensor 7.

The second check valve 13 has the function of preventing the fluid of the flow line 1 to enter the high pressure chamber 11 through the output port of the device for analyzing light chemical compounds AD. The second check valve 13 is for example a ball check valve that is known in the art.

The sensor is a sensor that is able to measure light or volatile chemical compounds in the gas portion of the sample. Typical light chemical compounds that may be measured are H₂S, CO₂, Hg, Radon, low weight alkane, etc... Typical operating principle of the sensor may be based on gas chromatography, optical absorption/transmission spectroscopy, etc...

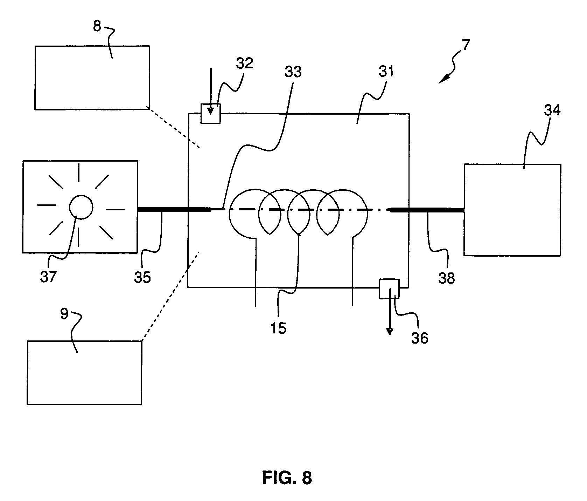

As an example, FIG.8 depicts a functional diagram illustrating an embodiment of a sensor 7 under the form of an optical absorption/transmission spectroscopy sensor. The absorption/transmission spectrometer comprises a housing defining an optical cell 31, an inlet port 32, an output port 36, a light source 37, a detector 34, and wave guides 35 and 38. The sensor 7 is coupled to a pressure sensor 8 and a temperature sensor 9. The sensor 7 may further comprise a heating coil 15.

The housing defines an optical cell 31 in which the sample may be analyzed. The sample flows from the low pressure chamber 4 into the optical cell through the inlet port 32, and flows out of the optical cell through the output port 36.

The light source 37 can be any kind of electromagnetic wave emitter operating at wavelengths from infra-red to ultra-violet. The light source 37 may be a light emitting diode, or an incandescent lamp, or a Xenon lamp, etc...The light source 37 is coupled to the optical cell though a first waveguide 35.

The detector 34 is any detector capable of providing dispersive or non dispersive spectroscopy measurement. The detector 34 is coupled to the optical cell though a second waveguide 38.

The waveguides 35 and 38 can be made out of sapphire rod, or silica based optical fibers. They can also be made of a combination of optical fibers and chemically resistant windows provided in the walls of the optical cell, e.g. sapphire windows.

The electromagnetic waves travel through the optical cell from the light source to the detector via a straight optical path 33. The sample of fluid flows through the optical path 33 from the inlet port 32 to the output port 36. The electromagnetic waves traveling through the optical cell are modified by the compounds present in the sample of fluid. The detection of these modifications (absorption/transmission spectrum) enables detecting the compounds and their respective quantities in the sample of fluid. The optical sensor may also comprise appropriate filters (not shown).

The pressure sensor 8 measures the pressure of the sample in the optical cell 31. It allows controlling the sample pressure in the sensor via the low pressure piston 5. Advantageously, the dead volume associated with the pressure sensor 8 is smaller than the total volume of the low pressure chamber 11.

The temperature sensor 9 measures the temperature of the sample in the optical cell 31. It allows controlling the sample temperature in the sensor via the heating coil 15.

As an example, a sensor 7 for H₂S measurement under the form of an optical absorption spectroscopy sensor may have the following characteristics. The optical cell optical path length may be of the order of 2 cm. The optical path cross section may be of the order of 500 µm. The light source may be a pulsed Xenon light source, providing an electromagnetic wave having a spectrum with ray around 200 nm. The detector operates in the 200 nm region.

FIG. 9 is a diagram illustrating the sequence of steps of the method for analyzing light chemical compounds of the invention. The sequence of operations is controlled by the controller 14.

In a first step S1, the injector 3 flashes a sample of fluid SP in the low pressure chamber 4. Thus, the sample of fluid SP is taken from the flow-line 1. The sample of fluid is depressurized so that light compounds of the sample become gaseous.

In a second step S2, the low pressure piston 5 moves until the pressure measured in the chamber of the sensor 7 is optimal.

In a third optional step S3, the heating coil 15 may heat the fluid sample in the sensor chamber until the temperature measured in the chamber of the sensor 7 is optimal.

In a fourth step S4, at least one characteristic related to at least one compounds of the fluid sample is measured by the sensor 7.

When the measurements are taken, the sample pressure and temperature are continuously controlled. Advantageously, the pressure is continuously adjusted from a few bars to a thousand bars while the temperature is maintained substantially constant, for example around the well temperature. The control of the optimal pressure and temperature enables adjusting the optical density of the fluid in the optical cell by a factor 1 to 100 while having a sensor with a constant optical cell length. On the one hand, it is possible to analyze light compound having a high concentration in the sample, thus importantly absorbing the electromagnetic wave even at a low pressure (few bars). On the other hand, it is also possible to analyze light compound having a low concentration in the sample, thus weakly absorbing the electromagnetic wave at a low pressure (few bars) but importantly absorbing the electromagnetic wave at a high pressure (thousand bars). Therefore, adjusting the pressure from a low value to a high value while analyzing the light compounds of the sample with the sensor enables sweeping a range of concentration of the light compound, and determining the best value, namely the light compound concentration having a sufficiently low measurement error. As a consequence, the measurement dynamic range (ratio between the highest and lowest concentrations that can be measured) is important.

In a fifth step S5, the low pressure piston 5 is fully extended. Thus, the sample of fluid is drain out of the chamber of the sensor 7 into the high pressure chamber 11.

In a sixth step S6, the high pressure piston 12 is fully extended. Thus, the sample of fluid that has been analyzed is discarded from the analyzing device. Advantageously, all these operations can be performed downhole.

Then, in a seventh step S7, the low pressure 5 and high pressure 12 pistons are retracted. The analyzing device is ready for analyzing a new sample of fluid.

### FINAL REMARKS

Though the invention has been described in relation with a particular application of the analyzing device to an onshore hydrocarbon well location, the invention may also apply to offshore hydrocarbon well location. Further, the invention is not limited to oilfield application as those versed in the art will recognize that the invention may apply in other application where light compounds contained in fluid needs to be analyzed, e.g. sewage application, chemical industry, etc....

The drawings and their description hereinbefore illustrate rather than limit the invention.

Any reference sign in a claim should not be construed as limiting the claim. The word "comprising" does not exclude the presence of other elements than those listed in a claim. The word "a" or "an" preceding an element does not exclude the presence of a plurality of such element.

## Claims

1. An analyzing device to analyze light chemical compounds comprises:
- an injector (3) to inject a fluid sample from a flow-line (1) in the analyzing device,
- a low pressure chamber (4) comprising a low pressure piston (5), the low pressure chamber (4) receiving the fluid sample from the injector (3),
- a high pressure chamber (11) comprising a high pressure piston (12), the high pressure chamber (11) receiving the fluid sample from the low pressure chamber (4),
- an analyzing sensor (7) coupled to one or more control sensors (8, 9), and receiving the fluid sample from the high pressure chamber (11), and
- a controller (14) receiving measurements from the analyzing sensor (7) and the control sensors (8, 9) and commanding operations of the injector (3), the low pressure piston (5), and the high pressure piston (12) such that the low pressure piston (5) pushes the fluid sample toward the analyzing sensor (7) and the high pressure chamber (11) in sweeping a determined pressure range encompassing an optimal pressure, the high pressure piston (12) pushes the fluid sample back to the flow line (1) after the fluid sample has been transferred to the high pressure chamber (11).

2. An analyzing device according to claim 1, wherein the analyzing sensor (7) further comprises a temperature regulator (15), operations of which being commanded by the controller (14).

3. An analyzing device according to claim 1 or 2, wherein the injector comprises a sliding valve (20) fitted in a body (21), the sliding valve (20) comprising a sampling chamber (23), at least one seal (24, 25, and 26) and an output port (27), the body being coupled to the flow-line (1) and comprising a bypass groove (22).

4. An analyzing device according to claim 3, wherein the sampling chamber (23) comprises walls that are textured such as to enhance the trapping of liquid in the sampling chamber (23).

5. An analyzing device according to any one of the preceding claims, wherein the device further comprises a first check valve (10) between the high pressure chamber (11) and the sensor (7) to prevent the fluid sample from uncontrolled exit of the high pressure chamber (11).

6. An analyzing device according to any one of the preceding claims, wherein the device further comprises a second check valve (13) between the high pressure chamber (11) and the flow-line (1) to prevent the fluid of the flow-line (1) to enter the high pressure chamber (11) through an output port (45).

7. An analyzing device according to any one of the preceding claims, wherein the device further comprises a filter (6) between the high pressure chamber (11) and the analyzing sensor (7) to trap at least a portion of a liquid phase contained in the fluid sample.

8. An analyzing device according to any one of the preceding claims, wherein the sensor (7) is an optical absorption/transmission spectroscopy sensor comprising a housing defining an optical cell (31), an inlet port (32), an output port (36), and an optical path (33) between a light source (37) and a detector (34) coupled to the optical cell by respective wave guides (35, 38).

9. An analyzing device according to any one of the preceding claims, wherein the sensor (7) is a gas chromatography sensor.

10. An analyzing device according to any one of the preceding claims, wherein the control sensors comprises a pressure sensor (8) and a temperature sensor (9).

11. An analyzing device according to any one of the preceding claims, wherein the temperature regulator is a heating coil (15) to adjust the temperature within the analyzing sensor (7) at an optimal temperature value.

12. An analyzing device according to any one of the preceding claims, wherein the high pressure piston (12) has a smaller cross section than the low pressure piston (5).

13. A method to perform downhole analysis of light chemical compounds, comprising the steps of:
- flashing a fluid sample from a flow-line (S1) in order to depressurize the fluid sample so that light compounds of the fluid sample become gaseous, and transferring the fluid sample to a low pressure chamber,
- sweeping a range of pressure in the low pressure chamber at least until the pressure measured by an analyzing sensor is optimal (S2),
- measuring at least one characteristic related to at least one compound of the fluid sample (S4) while continuously controlling the fluid sample pressure and temperature,
- compressing the fluid sample in a high pressure chamber (S5), and
- discarding the fluid sample to the flow-line (S6).

14. An analyzing method according to claim 13, wherein the method further comprises heating the fluid sample in the low pressure chamber until the temperature measured in the low pressure chamber is optimal (S3).

15. An analyzing method according to claim 13 or 14, wherein the pressure is adjusted from a low value to a high value while measuring at least one characteristic related to at least one compound of the fluid sample such as a range of concentration of the compound is swept, the optimal pressure corresponding to a light compound concentration associated with the lowest measurement error.

16. An analyzing method according to claim 13, wherein the analyzing sensor is in communication with the high pressure chamber.
